# EUROPEAN PATENT APPLICATION

(11) **EP 2 502 640 A1**
(43) Date of publication of application: **26.09.2012**
(21) Application number: 11158960.2
(22) Date of filing: 21.03.2011
(51) Int. Cl.: A61M 1/06

(54) **Breast pump**

(71) Applicant: Koninklijke Philips Electronics N.V., 5621 BA Eindhoven (NL)
(72) Inventor: Darnell, Ian, P., 5600 AE, Eindhoven (NL); Cook, Graham, T., 5600 AE, Eindhoven (NL)
(74) Representative: Damen, Daniel Martijn

(57) **Abstract**

A breast pump comprising a funnel (2) and at least two cone-shaped breast pump inserts (1) interchangeably mountable in said funnel (2) is disclosed. The breast pump inserts (1) each have a central aperture (10,12) therein through which a nipple protrudes when a breast is received in the funnel (2). The diameter of the aperture (10,12) in each breast pump insert (1) is different. A breast pump funnel (2) or insert (1) having a textured finish on a breast interface surface is also disclosed.

## Description

### FIELD OF THE INVENTION

The present invention relates to a breast pump. It also relates to a modified breast pump insert to replace a standard breast pump insert supplied with a breast pump such that the breast pump can be used interchangeably with the standard or modified breast pump insert and, to a breast pump insert kit.

The invention also relates to a breast pump or breast pump insert that provides an enhanced level of comfort for a user.

### BACKGROUND OF THE INVENTION

Breast pumps are well known devices for extracting milk from a breast of a user. A breast pump may be used if the baby or infant is not itself able to extract milk from the breast, or if the mother is separated from the baby or infant and is to be fed with breast milk by someone else. Breast pumps typically comprise a rigid funnel connected to a vacuum pump having a container for collecting the milk.

The funnel of a breast pump is the interface between the user's breast and nipple with the pump and so the sizing of the funnel is critical to maintaining the user's comfort whilst using the device. It is also important to ensure that the vacuum seal between the breast and the insert is maintained for optimal pumping. A problem with a conventional breast pump is that it has a funnel of fixed size, so it can only cater for a limited range of breast and nipple sizes. However, if the funnel is too small relative to the nipple, the nipple tends to fill the available space inside the funnel and is likely to touch on the sides of the funnel, resulting in chafing, friction and discomfort as negative pressure generated during use of the pump draws the nipple into the funnel. On the contrary, if the funnel is too large relative to the nipple, then there will be more dead space inside the funnel which will reduce the efficiency of the pump system and limit the negative pressure achievable. It also introduces the possibility that the nipple will be pulled deeper into the pump and that the skin on areola or breast area surrounding the nipple will be subjected to chafing.

Research has shown that nipple diameter and length varies throughout the population and across different geographic regions and also that the sizing of the nipple can be different before, during and after expressing. Therefore, as fit is an important consideration when attempting to achieve maximum comfort for a user, a breast pump that is capable of accommodating a wide range of breast and nipple sizes is desirable.

It is known to provide a breast pump body with a removable funnel that may be replaced with another funnel of a different size. However, removable funnels are generally made from a hard plastic material and do not generally offer the user with an enhanced level of comfort whilst using such a device.

It is also known to provide a soft elastomer insert that may be disposed within a rigid funnel of a breast pump and which is designed to adapt to the contour of the breast so as to provide comfort and a vacuum seal necessary for operating the pump. The resilience and compliance of such an insert helps to provide a vacuum and milk seal around the user's breast and also reduces friction on the breast and/or nipple when the negative pressure draws the breast and nipple in a direction into the pump. A separate cushion or insert also allows for the inclusion of a pressure ducting system on the cushion's rear surface which faces the funnel. A pressure ducting system may include a system of 'petals' that can move in and out to palpate the breast. These petals can be formed from a plurality of regions spaced around the circumference of the insert to define a void or reservoir. The wall thickness in the region of the petals may be less than the remainder of the insert so that they deform more easily in response to the generation of a negative pressure when the pump is in use.

A cushion or insert may be formed from silicon or thermoplastic elastomer (TPE) which, in addition to providing an enhanced level of comfort, can also provide a warmer feeling to the breast.

Although an insert may improve the comfort for a user, breast pumps equipped with an insert still suffer from the problem that the insert will only accommodate a relatively small range of breast and/or nipple sizes resulting in a poor fit between the breast and/or the nipple with the insert for a relatively large number of breastfeeding women, causing discomfort and poor vacuum pressure generation.

### SUMMARY OF THE INVENTION

The present invention seeks to overcome or substantially alleviate the aforementioned problems.

Accordingly, the present invention provides a breast pump comprising a funnel and at least two funnel-shaped breast pump inserts interchangeably mountable in said funnel, each of said breast pump inserts having a central aperture therein through which a nipple extends when a breast is received therein, wherein the diameter of the aperture in each breast pump insert is different.

Preferably, each breast pump insert comprises a wall having a thickness. The thickness of said wall in a region of the aperture is different for each insert.

Each breast pump insert may have an outer diameter in the region of the aperture that is defined by the sum of twice the wall thickness and the diameter of the aperture.

Preferably, the outer diameter in the region of the aperture is substantially constant for all breast pump inserts.

In a preferred embodiment, each breast pump insert comprises a frustoconical section that tapers to a substantially cylindrical section. In this embodiment, the aperture is formed at an end of the substantially cylindrical section.

Preferably, said region of the aperture is defined by the substantially cylindrical section such that the thickness of said wall forming said substantially cylindrical section is different for each insert.

According to the invention, there is also provided a modified breast pump insert to replace a standard breast pump insert supplied with a breast pump such that said breast pump can be used interchangeably with the standard or modified breast pump insert, each of said breast pump inserts having a central aperture therein through which a nipple extends when a breast is received therein and an overall outer diameter in a region of the aperture, wherein the diameter of the aperture in the modified breast pump insert is different to the diameter of the aperture in the standard breast pump insert, said overall outer diameter of the breast pump insert in the region of said aperture remaining the same in both the modified and standard breast pump inserts irrespective of the difference in the diameter of the aperture between said inserts.

According to the invention, there is also provided a breast pump insert kit comprising at least two funnel-shaped breast pump inserts interchangeably mountable in a funnel of a breast pump, each breast pump insert having a central aperture therein through which a nipple extends when a breast is received therein, wherein the diameter of the aperture in each breast pump insert is different.

Preferably, each breast pump insert comprises a wall having a thickness, and wherein the thickness of said wall in a region of the aperture is different for each insert.

Each breast pump insert may have an outer diameter in the region of the aperture defined by the sum of twice the wall thickness and the diameter of the aperture. Preferably, the outer diameter of all the breast pump inserts is the same.

In one embodiment, each breast pump insert comprises a frustoconical section that tapers to a substantially cylindrical section. The aperture is then formed at an end of the substantially cylindrical section. Preferably, the length of the cylindrical section remains constant across all the breast pump inserts, despite the change in diameter of the aperture.

The region of the aperture may be defined by the substantially cylindrical section such that the thickness of said wall forming said substantially cylindrical section is different for each insert.

According to the invention, the breast pump insert kit may also include a breast pump according to the invention.

It has been determined that a preferred setting for the let-down of breast milk is one where the woman's breast is warm and she feels relaxed. Many women hold a warm flannel to the breast before expressing and also create a comfortable environment in order to relax and to aid the process of milk let-down.

Using a breast pump requires the funnel, or a breast pump insert, to make contact with the breast. However, the plain engineering materials and finishes for the surface of the funnel or insert that touches the breast have been perceived as being cold and hard by the user which can potentially inhibit the ease of milk let-down or initially just feel cold and uncomfortable as the user starts their pumping session.

Furthermore, it has been found that silicon parts with polished surfaces can feel sticky to the touch, feely clammy, and pick up dirt and lint, giving the perception of being dirty or unhygienic.

The present invention also seeks to overcome or alleviate the aforementioned problems. This aspect of the invention may be independent to the previous aspect of the invention referred to above or, both aspects may be combined.

According to the present aspect of the invention, there is provided a breast pump having a funnel to receive a breast of a user, wherein a surface of said funnel that comes into contact with a breast received in said funnel has a textured surface finish.

According to the invention, there is also provided a breast pump insert positionable within the funnel of a breast pump to receive a breast of a user, wherein a surface finish of said breast pump insert that comes into contact with a breast received in said insert has a textured surface finish.

The surface finish may be created on the funnel or on a breast pump insert during the manufacturing process through virtue of the mould-tool surface. This creates an irregular non-smooth surface that may have an arithmetical mean roughness (Ra) in the order of single digits to tens of digits that reduces the overall surface contact between the user's skin and the breast pump. The arithmetical mean roughness can be between 1 and 50Ra or even 1 to 3 Ra.

The surface texture reduces the overall surface contact between the user's skin and the breast pump. This results in reduced energy transfer between the skin and insert or funnel, making it seem less cold than it is. Furthermore, the small air pockets trapped in the undulations create a heat isolation layer that results in a warmer and more pleasant overall sensation compared to the colder sensation when using a pump with a smooth surface at the breast interface.

A textured surface also makes it less sticky, which improves the user's perception of the feel, reducing the tendency for it to feel clammy. It also picks up much less dirt and lint compared to a smooth silicon surface.

It will be appreciated that a textured surface finish can be applied to the whole or only part of the surface of a funnel or a breast pump insert that may come into contact with a user's breast. Preferably, the textured surface finish is applied to the entire inner surface of a breast pump insert or, if no insert is to be used, the entire conical shaped portion of a breast pump funnel may have a textured surface. However, it will be appreciated that the textured surface may be applied in a series of repeated patterns or to sections of the surface of the insert or to the funnel.

In one embodiment, the insert or funnel may have a smooth annular region at its mouth or periphery so that the textured surface covers an inner portion of the funnel or insert. This improves the seal between the funnel or insert and the user's breast whilst still maintaining the benefits of a textured surface.

### BRIEF DESCRIPTION OF THE DRAWINGS

Preferred embodiments will now be described, by way of example only, with reference to the accompanying drawings, in which:
Figures 1A and 1B show perspective views of a two inserts according to one embodiment of the present invention;
Figure 2A shows a cross-sectional side elevation of a breast pump body fitted with the breast pump insert of Figure 1A; and
Figure 2B shows a cross-sectional side elevation of a breast pump body fitted with the breast pump insert of Figure 1B.

### DETAILED DESCRIPTION OF THE EMBODIMENTS

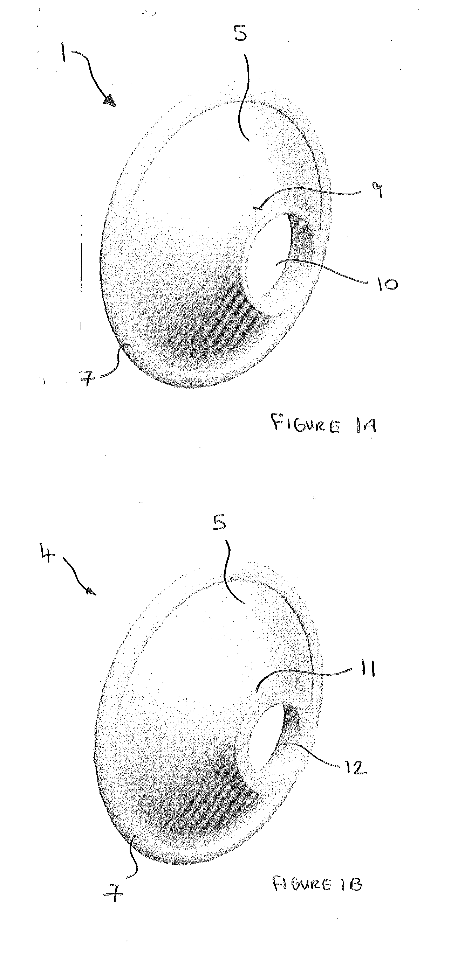

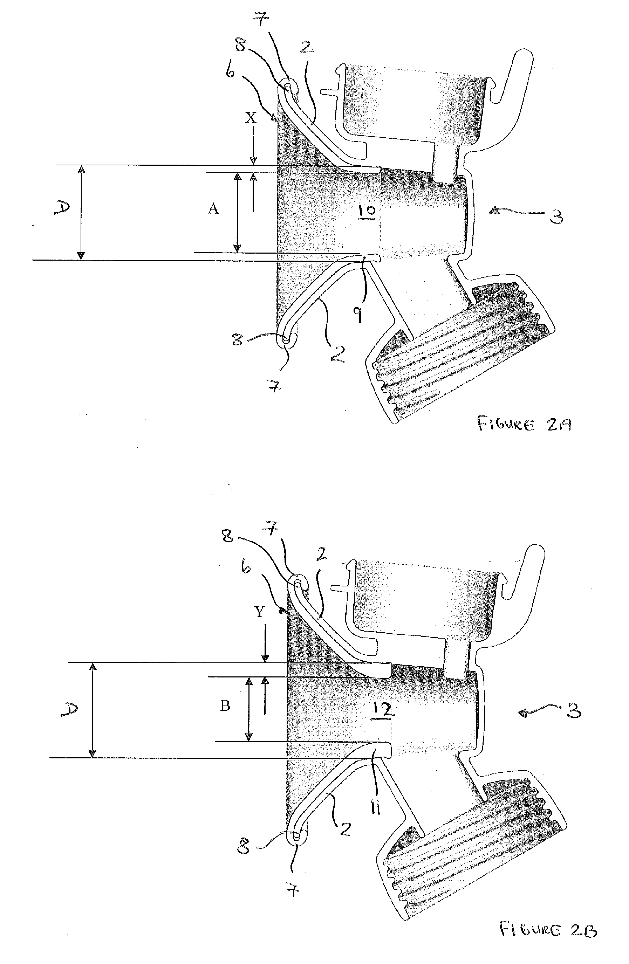

Referring now to the drawings, Figures 1A shows a first breast pump insert 1. The insert 1 may be disposed in a funnel 2 of a breast pump body 3 for extracting milk, as shown in Figure 2A.

Figure 1B shows a second breast pump insert 4. The insert 4 may also be disposed in a funnel 2 of a breast pump body 3 for extracting milk, as shown in Figure 2B, in place of the insert 1 shown in Figure 1A.

The breast pump body 3, or at least the funnel 2 of the breast pump body 3, shown in Figures 2A and 2B may have identical dimensions.

Each of the breast pump inserts 1,4 have a generally frustoconical shaped wall 5 having a mouth area 6 through which a breast is inserted into the insert 1,4 when the insert 1,4 is located within a funnel 2 of the breast pump body 3. The wall 5 has a peripheral lip 7 around the mouth 6 that locates around the outer extremity 8 of the funnel 2 to releasably attach the insert 1,4 to the funnel 2.

The wall 5 of breast pump insert 1 tapers to a short cylindrical section 9 that terminates in an aperture 10. Similarly, the wall 5 of breast pump insert 4 tapers to a short cylindrical section 11 that terminates in an aperture 12. The user's nipple protrudes through the aperture 10,12 of the breast pump insert 1,4 when one of said inserts 1,4 is received within the funnel 2 of the breast pump body 3 and a breast is placed within the insert 1,4 through the mouth 6.

The short cylindrical section 9, 11 of each breast pump insert 1,4 has an outer, overall diameter (as indicated by dimension "D" in Figures 2A and 2B), that is substantially identical. However, the wall thickness (indicated by dimension "X" in Figure 2A and dimension "Y" in Figure 2B) is different. More specifically, the wall thickness "X" is smaller than the wall thickness "Y". Preferably, the overall outer geometry of the breast pump inserts 1,4 are identical, in addition to the outer diameter of the short cylindrical section 9,11.

As a result of the difference in wall thickness and as there is no alteration in the overall diameter of the short cylindrical section 9,11 between inserts 1,4, the diameter of the apertures 10,12 are different to each other. In particular, the diameter of the aperture 10 in Figure 2A (indicated by dimension A) is greater than the diameter of the aperture 12 in Figure 2B (indicated by dimension B).

In a preferred embodiment, the length of the cylindrical sections 9,11 of the inserts remains the same despite the change in diameter of the aperture 10,12. Most preferably, the overall length of the breast pump inserts is the same.

As at least the outer diameter of the short cylindrical section 9,11 of each breast pump insert 1,4 is the same, either breast pump insert 1,4 may be used interchangeably with the same breast pump 3 or funnel 2 and no modification to the pump body 3 or funnel 2 is required when the breast pump insert 1 is changed for the breast pump insert 4, and vice-versa. A user may select a preferred insert 1,4 depending on their breast and/or nipple size and/or depending on which insert provides them with the most comfort during pumping, and fit that insert 1,4 to the funnel 2 of their pump body 3, without any replacement of, or modification to, their pump body or funnel 2.

Each of the breast pump inserts 1,4 are formed from a compressible and resilient material such that they easily deform and adapt to the contour of the breast. Preferably, each breast pump insert 1,4 is formed from a silicone gel, silicone rubber material or thermoplastic elastomer (TPE), although it will be appreciated that any material having resilient, deformable properties, that will provide comfort to the user and achieve a vacuum by adapting to the shape of the breast can be used.

The invention caters for different nipple sizes by providing a number or range of breast pump inserts, each of which have a different diameter aperture in the breast pump insert through which the nipple protrudes or extends.

Despite the change in aperture diameter across the range of inserts, the outer dimension of the insert, at least in the region of the aperture, remains constant, thereby enabling any of the inserts to be fitted to the same breast pump body or funnel, without modification.

It will be appreciated that each insert may be provided with integral petals or other features to massage the breast during pumping.

Although the invention has been described with reference to two breast pump inserts 1,4, it will be appreciated that any number or range of inserts may be provided, each of which have different aperture diameters, but the same overall or outer diameter, so that they will all fit the same pump body 3 or funnel 2.

It is envisaged that a breast pump may be provided with two or more inserts each having a different aperture diameter so that a user may select the most appropriate insert for maximum comfort. However, it is also envisaged that one or more modified breast pump inserts(s) may be made available to replace a standard breast pump insert that has previously been supplied with a breast pump. The same breast pump can then be used interchangeably with the standard or modified breast pump insert as the overall outer diameter of the breast pump insert in the region of said aperture remains the same in both the modified and standard breast pump inserts irrespective of the difference in the diameter of the aperture between said inserts. It is also envisaged that a breast pump insert kit may be provided, i.e. a set of two or more breast pump inserts, each of which have a different aperture diameter but the same overall outer diameter, at least in the region of the aperture. Each of the inserts of the kit may then be used interchangeably with the same breast pump.

In connection with the second aspect of the invention referred to above, it will be appreciated that one or both of the inserts of the first aspect of the invention may be provided with a textured surface. The present invention also includes within its scope a single breast pump insert having a textured breast interface contact surface, as well as a funnel for a breast pump having such a surface.

The textured surface may cover all or part of the funnel or insert. For example, there can be a series of repeated patterns extending around the circumference of the insert or cushion each of which have a surface texture. In one preferred embodiment, there is a ring of smooth or untextured surface extending around the circumference of the insert or funnel to ensure that a proper seal is formed with a breast inserted therein. The smooth region can be at the mouth of the insert or funnel, i.e. at the end or outer border where the breast is inserted or, it can be spaced inwardly so as to divide the textured surface into two regions separated by said ring.

If an insert is provided with a series of petals, as previously mentioned above, the surface texture could be associated with those petals, i.e. the surface texture may only be on the surface of the insert forming the petals. Alternatively, it could surround the petals.

The textured surface may be formed from a surface having an arithmetical mean roughness (Ra) in the order of single digits to tens of digits that reduces the overall surface contact between the user's skin and the breast pump. However, more preferably the arithmetical mean roughness is between 1 and 50Ra or even 1 to 3 Ra.

The invention has been described with reference to preferred embodiments only. Modifications and alterations to the embodiments falling within the scope of the appended claims are included within the scope of protection.

## Claims

1. A breast pump comprising a funnel and at least two funnel-shaped breast pump inserts interchangeably mountable in said funnel, each of said breast pump inserts having a central aperture therein through which a nipple extends when a breast is received therein, wherein the diameter of the aperture in each breast pump insert is different.

2. A breast pump according to claim 1, wherein each breast pump insert has an outer diameter in a region of the aperture and comprises a wall having a thickness, and wherein the thickness of said wall in a region of the aperture is different for each insert.

3. A breast pump according to claim 2, wherein the outer diameter in the region of the aperture is defined by the sum of twice the wall thickness and the diameter of the aperture.

4. A breast pump according to claim 2 or claim 3, wherein the outer diameter of the breast pump inserts is substantially the same.

5. A breast pump according to any of claims 2 to 4, wherein each breast pump insert comprises a frustoconical section that tapers to a substantially cylindrical section, said aperture being formed at an end of the substantially cylindrical section.

6. A breast pump according to claim 5, wherein said region of the aperture is defined by the substantially cylindrical section such that the thickness of said wall forming said substantially cylindrical section is different for each insert.

7. A modified breast pump insert to replace a standard breast pump insert supplied with a breast pump such that said breast pump can be used interchangeably with the standard or modified breast pump insert, each of said breast pump inserts having a central aperture therein through which a nipple extends when a breast is received therein and an overall outer diameter in a region of the aperture, wherein the diameter of the aperture in the modified breast pump insert is different to the diameter of the aperture in the standard breast pump insert, said overall outer diameter of the breast pump insert in the region of said aperture remaining the same in both the modified and standard breast pump inserts irrespective of the difference in the diameter of the aperture between said inserts.

8. A breast pump insert kit comprising at least two funnel-shaped breast pump inserts interchangeably mountable in a funnel of a breast pump, each breast pump insert having a central aperture therein through which a nipple extends when a breast is received therein and a wall having a thickness, wherein the diameter of the aperture, and the thickness of the wall in a region of the aperture in each breast pump insert is different.

9. A kit according to claim 8, wherein each breast pump insert has an outer diameter in the region of the aperture defined by the sum of twice the wall thickness and the diameter of the aperture, the outer diameter of each breast pump insert being substantially the same.

10. A kit according to claims 8 or 9, wherein each breast pump insert comprises a frustoconical section that tapers to a substantially cylindrical section, said aperture being formed at an end of the substantially cylindrical section.

11. A breast pump according to claim 10, wherein said region of the aperture is defined by the substantially cylindrical section such that the thickness of said wall forming said substantially cylindrical section is different for each insert.

12. A kit according to any of claims 8 to 11, including a breast pump according to any of claims 1 to 6.

13. A breast pump having a funnel to receive a breast of a user, wherein a surface of said funnel that comes into contact with a breast received in said funnel has a textured surface finish.

14. A breast pump insert positionable within the funnel of a breast pump to receive a breast of a user, wherein a surface finish of said breast pump insert that comes into contact with a breast received in said insert has a textured surface finish.

15. A breast pump according to claim 13 or a breast pump insert according to claim 14, wherein the textured surface finish has an arithmetical mean roughness (Ra) in the order of single digits to tens of digits that reduces the overall surface contact between the user's skin and the breast pump.
